(19) Europäisches Patentamt
European Patent Office
Office européen des brevets

(11) Numéro de publication: **0 530 085 B1**

(12) **FASCICULE DE BREVET EUROPEEN**

(45) Date de publication de fascicule du brevet: **15.03.95**

(51) Int. Cl.6: **A61K 7/48**, A61K 7/02, A61K 7/021

(21) Numéro de dépôt: **92402318.7**

(22) Date de dépôt: **20.08.92**

(54) **Composition cosmétique pour le maquillage de la peau contenant au moins une cire de silicone et son procédé de préparation.**

(30) Priorité: **30.08.91 FR 9110792**

(43) Date de publication de la demande: **03.03.93 Bulletin 93/09**

(45) Mention de la délivrance du brevet: **15.03.95 Bulletin 95/11**

(84) Etats contractants désignés: **DE FR GB IT**

(56) Documents cités:
**EP-A- 0 106 762**
**FR-A- 2 528 699**

(73) Titulaire: **L'OREAL**
**14, Rue Royale**
**F-75008 Paris (FR)**

(72) Inventeur: **Bara, Isabelle**
**68, avenue d'Italie**
**F-75013 Paris (FR)**
Inventeur: **Mellul, Myriam**
**17, allée du Parc de la Bièvre**
**F-94240 l'Hay-les-Roses (FR)**

(74) Mandataire: **Peuscet, Jacques**
**SCP Cabinet Peuscet et Autres,**
**68 Rue d'Hauteville**
**F-75010 Paris (FR)**

**EP 0 530 085 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne une composition cosmétique pour le maquillage de la peau contenant au moins une cire de silicone sous forme de dispersion dans une phase aqueuse, cette composition étant stable sans qu'il soit nécessaire d'y introduire un agent tensioactif.

Les produits de maquillage de la peau sont souvent des émulsions d'une phase grasse dans une phase aqueuse ; il faut évidemment qu'une telle émulsion soit stable au cours du temps pour être acceptable sur le plan commercial. Généralement on assure la stabilité desdites émulsions en y incorporant des agents tensioactifs. Malheureusement, la présence d'agents tensioactifs est souvent une source d'inconfort ou d'irritation de la peau et il est souhaitable de l'éviter.

Dans les compositions de maquillage de la peau tels que rouges à lèvres et fards à paupières, il est souvent intéressant d'utiliser, comme constituant de la phase grasse, une cire et non pas une huile, de façon à obtenir des produits plus consistants que l'on peut appliquer sous forme de pain ou de bâton. On souhaite, en particulier, utiliser des cires de silicone car lesdites cires possèdent des propriétés cosmétiques intéressantes : bonne facilité d'étalement, pouvoir filmogène important, brillance et résistance à l'eau. Mais jusqu'à présent, il était pratiquement impossible de préparer avec des cires, des émulsions sans tensioactif qui soient stables au cours du temps et contiennent un fort pourcentage de cire(s). Au demeurant, avec les cires de silicone, même la présence de tensioactifs ne résolvait pas le problème : il se produisait généralement, dès que le pourcentage de cire de silicone était élevé, une coalescence de la cire de silicone et on obtenait des émulsions grossières et peu stables, qui ne pouvaient être utilisées pour le maquillage de la peau.

On a déjà proposé dans la demande de brevet JP-02-019310, une composition de maquillage constituée par une dispersion huile dans l'eau, dont la stabilité est assurée grâce à l'incorporation, dans la phase aqueuse, de polymères acryliques réticulés. De même, dans EP-A-268 164, on a décrit la stabilisation d'une émulsion huile dans l'eau à l'aide de polymères acryliques. Dans la phase grasse de ces deux compositions de maquillage, on ne trouve néanmoins que des huiles et jamais de cires de silicone ; et il s'est avéré, en effet, que les cires de silicone ne sont pas stabilisées par les systèmes décrits.

La présente invention a pour but d'obtenir, dans une phase aqueuse, une dispersion stable d'au moins une cire de silicone, ladite dispersion ayant une forte teneur en cire de silicone, sans qu'il soit nécessaire d'ajouter des agents tensioactifs. Selon l'invention, ce but est atteint en associant à la (aux) cire(s) de silicone, dans la phase aqueuse deux polymères acryliques particuliers.

La présente invention a donc pour objet une composition cosmétique pour le maquillage de la peau contenant au moins une cire de silicone, caractérisée par le fait que la (les) cire(s) de silicone est (sont) dispersée(s) dans une phase aqueuse contenant deux types de polymère(s) acrylique(s) hydrosoluble(s), le premier type de polymère(s) étant constitué par au moins un copolymère d'un acide monoéthylénique en $C_3$ - $C_6$ ou de son anhydride avec un ester arylique à longue chaîne et le second type de ces polymère(s) étant constitué par au moins un homo- ou copolymère d'acide acrylique ou un de ses sels, les copolymères d'acide acrylique avec un ester d'acide acrylique à longue chaîne et leurs sels étant exclus.

On a trouvé, selon l'invention que la présence de ces deux types de polymères acryliques était nécessaire pour obtenir, dans une phase aqueuse, une dispersion stable de cire(s) de silicone, contenant plus de 10 % en poids de cire de silicone et qu'elle permettait d'obtenir des dispersions stables contenant jusqu'à 60 % en poids de cire(s) de silicone.

Le premier type de polymère(s) acrylique(s) hydrosoluble(s), qui sera appelé par la suite "polymère modifié", est décrit dans EP-A-O 268 164. Ce polymère modifié est un copolymère, éventuellement réticulé, d'un acide carboxylique monoéthylénique comportant 3 à 6 atomes de carbone (ou de son anhydride) et d'un ester acrylique à chaîne longue. Dans ce copolymère, la proportion d'acide monomère est, de préférence, de 90 à 98 % en poids et la proportion d'ester monomère est, de préférence, de 10 à 2 % en poids.

L'acide monomère a pour formule :

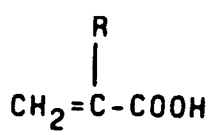

formule dans laquelle R représente H, un halogène, OH, un radical lactone ou lactame, un groupe -C = N, ou un radical alkyle, aryle, aralkyle, alkylaryle ou cycloaliphatique. Les monomères acides préférés sont

l'acide acrylique et l'anhydride maléique.

L'ester monomère a pour formule :

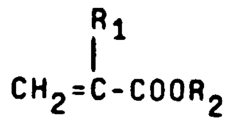

formule dans laquelle :

$R_1$ est H, méthyle ou éthyle et

$R_2$ est un radical aile en $C_8$ - $C_{30}$, oxyalkylène en $C_8$ - $C_{30}$ ou carbonyloxyalkylène en $C_8$ - $C_{30}$. Les radicaux alkyle en $C_{10}$ - $C_{22}$ sont préférés. Parmi les esters monomères que l'on préfère, on peut citer : les acrylates et méthacrylates de décyle, lauryle, stéaryle, béhényle et mélissyle.

Les polymères modifiés utilisés selon l'invention sont, au moins pour certains d'entre eux, distribués dans le commerce ; ils sont, par exemple, commercialisés sous la dénomination "PEMULEN" par la société "BF GOODRICH".

Le second type de polymère(s) acrylique(s) hydrosoluble(s) est un homo- ou copolymère d'acide acrylique, éventuellement réticulé ou un de ses sels, les copolymères d'acide acrylique et d'un ester d'acide acrylique et leurs sels qui font partie du premier type de polymères sont exclus ; ces polymères du second type sont, par exemple, ceux commercialisés sous la dénomination "CARBOPOL" par la société "BF GOODRICH", sous la dénomination "HOSTACERIN" (copolymère d'acrylate de sodium et d'acrylamide) ou sous la dénomination "PAS 5161" (copolymère d'acrylate d'ammonium et d'acrylamide) par la société "HOECHST".

Le premier type de polymère(s) ou "polymère modifié" est présent dans des proportions comprises entre 0,01 et 1 % en poids par rapport au poids total de la composition et, de préférence, entre 0,02 et 0,2 % en poids.

Le second type de polymère(s) est présent dans des proportions comprises entre 0,2 et 2 % en poids par rapport au poids total de la composition et, de préférence, entre 0,4 et 1,5 % en poids.

Les cires de silicone utilisées sont celles généralement utilisées en cosmétique. Parmi celles-ci, on peut plus particulièrement citer les organosiloxanes ayant la formule suivante :

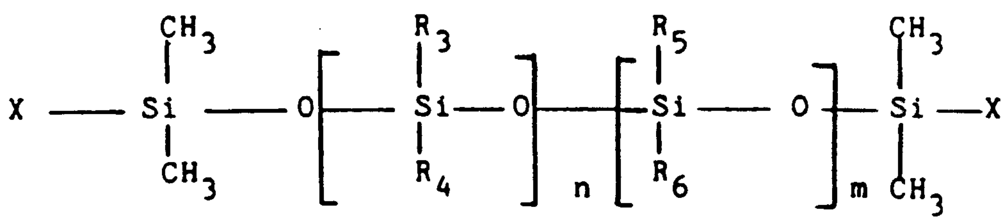

formule dans laquelle :

$R_3$, $R_5$ et $R_6$ sont indépendamment les uns des autres, un radical alkyle ou alkoxy en $C_1$ - $C_{30}$ ou un radical aryle en $C_6$ - $C_{30}$ ;

$R_4$ est un radical alkyle ou alkoxy en $C_2$ - $C_{36}$ ou un radical alkyle ou alkoxy en $C_2$ - $C_{36}$ substitué par un groupe ester, R4 pouvant également être un radical méthyle lorsque X est un radical alkyle ou alkoxy en $C_2$ - $C_{30}$ ou un ester en $C_2$ - $C_{30}$ ;

X est un radical alkyle ou alkoxy en $C_1$ - $C_{30}$ , un radical aryle en $C_6$ - $C_{30}$ ou un ester en $C_1$ - $C_{30}$ ;

n est un nombre entier compris entre 1 et 100.

m est 0 ou un nombre entier compris entre 1 et 100.

On peut citer par exemple l'alkyldiméthicone commercialisé sous la dénomination "ABIL WAX 9810" par la Société GOLDSCHMIDT, le dibéhénoxydiméthicone commercialisé sous la dénomination "ABIL WAX 2440" par la Société GOLDSCHMIDT ou le stéaroxydiméthicone commercialisé sous la dénomination "VP 1622" par la Société WACKER.

On peut également utiliser les organosiloxanes obtenus par action d'une cire naturelle, telle que la cire de Carnauba ou la cire d'abeilles, sur un squelette de silicone réactif.

La cire de silicone représente de 10 à 60 % en poids par rapport au poids total de la composition. Pour des quantités de cires inférieures à 10 %, il est possible d'obtenir des émulsions de cire(s) de silicone en

l'absence des deux types de polymère(s) de la présente invention ; pour des quantités supérieures à 60 % en poids, il n'est plus possible d'obtenir des émulsions stables au cours du temps.

La phase aqueuse de l'émulsion de cires de silicone peut contenir des polymères hydrosolubles autres que les deux types de polymère(s) acrylique(s) nécessaires pour la mise en oeuvre de l'invention. Ces autres polymères hydrosolubles peuvent être :

- des dérivés de protéines et, plus particulièrement, des dérivés de kératine tels que les hydrolysats de kératine et les kératines sulfoniques ;
- des dérivés de cellulose, tels que l'hydroxyéthylcellulose, l'hydroxypropylcellulose, la méthylcellulose, l'éthylhydroxyéthylcellulose , la carboxyméthylcellulose ;
- des polymères naturels tels que les gommes arabiques et les dérivés de xanthane ;
- les polyvinylpyrrolidones et
- l'alcool polyvinylique.

La phase aqueuse peut également contenir des alcools ou des polyols utilisés de façon générale dans les cosmétiques tels que l'éthanol, la glycérine, les polyglycérines ou tout autre ingrédient hydrosoluble.

La (les) cire(s) de silicone peut(vent) être mélangée(s) avec des cires classiques généralement utilisées en cosmétique telles que les cires animales, (cires d'abeilles, lanoline), les cires végétales (cire de Carnauba, cire de Candelilla), les cires minérales (paraffines, ozokérites, cires microcristallines) ou les cires synthétiques (cires de polyéthylène). Dans un tel mélange, la (les) cire(s) de silicone représente(nt), de préférence, au moins 20 % en poids par rapport au poids total des cires de la composition et au moins 10 % en poids par rapport au poids total de la composition.

La composition cosmétique peut également contenir des charges colorées et/ou des charges non colorées.

Les charges colorées sont des pigments minéraux, organiques et/ou nacrés.

On peut citer, à titre d'exemples de pigments minéraux, le dioxyde de titane (rutile ou anatase), éventuellement traité en surface, et codifié dans le Color Index (C.I.) sous la référence CI 77891, les oxydes de fer noir, jaune, rouge et brun codifiés sous les références CI 77499, 77492, 77491, le violet de manganèse (CI 77742), le bleu outremer (CI 77007), l'oxyde de chrome (CI 77288), l'hydrate de chrome (CI 77289), le bleu ferrique (CI 77510).

Les pigments organiques sont choisis, en particulier, parmi le noir de carbone, les pigments D et C red n° 19 (CI 45170), D et C red n° 9 (CI 15585), D et C red n° 21 (CI 45380), D et C orange n° 4 (CI 15510), D et C orange n° 5 (CI 45370), D et C red n° 27 (CI 15850), D et C red n° 6 (CI 15850), D et C yellow n° 5 (CI 45425), D et C yellow n° 6 (CI 15985), D et C red n° 30 (CI 73360), D et C red n° 3 (CI 45430) et les laques à base de carmin de cochenille (CI 75470).

Les pigments nacrés peuvent notamment être choisis dans le groupe formé par le mica recouvert d'oxyde de titane, l'oxychlorure de bismuth, le mica titane avec des oxydes de fer et le mica titane avec du bleu ferrique ou de l'oxyde de chrome.

Les pigments, lorsqu'ils sont utilisés, représentent généralement de 0,5 à 20 % en poids du poids total de la composition.

Les charges non colorées sont notamment choisies dans le groupe formé par :

- le talc, qui est un silicate de magnésium hydraté, utilisé sous forme de particules généralement de dimensions inférieures à 40 µm ;
- les micas, qui sont des aluminosilicates de compositions variées, qui se présentent sous la forme d'écailles ayant des dimensions de 2 à 200 µm, de préférence de 5 à 70 µm et une épaisseur de 0,1 à 5 µm, de préférence de 0,2 à 3 µm, ces micas pouvant être d'origine naturelle (par exemple muscovite, margarite, roscoelithe, lipidolite, biotite) ou d'origine synthétique ;
- l'amidon, en particulier l'amidon de riz ;
- le kaolin, qui est un silicate d'aluminium hydraté et se présente sous la forme de particules de forme isotrope ayant des dimensions généralement inférieures à 30 µm ;
- les oxydes de zinc et de titane, généralement utilisés sous forme de particules ayant des dimensions ne dépassant pas quelques micromètres (ou même inférieures à 1 µm dans le cas de l'oxyde de titane) ;
- le carbonate de calcium ;
- le carbonate ou l'hydrocarbonate de magnésium ;
- la cellulose microcristalline ;
- les poudres de polymères synthétiques, tels que le polyéthylène, les polyesters, (par exemple isophtalate ou téréphtalate de polyéthylène), les polyamides, les téflons et les poudres de silicones réticulées creuses ou pleines.

Les charges, lorsqu'elles sont utilisées peuvent représenter jusqu'à 40 % en poids par rapport au poids total de la composition.

Les pigments et les charges peuvent, de façon connue, être enrobés par des substances telles que des acides aminés, des silicones, des sels métalliques ou du collagène ou avoir subi un traitement permettant de modifier leur état de surface.

Les compositions selon l'invention peuvent également contenir au moins un composé inerte ou actif généralement utilisé dans les compositions cosmétiques, notamment pris dans le groupe formé par des adoucissants, des conservateurs, des séquestrants, des parfums, des huiles, y compris des huiles de silicone, des agents alcanisants ou acidifiants pour ajuster le pH de la composition, ainsi que des vitamines, des acides aminés ou autres actifs.

La présente invention a également pour objet un procédé de fabrication de la composition selon l'invention dans lequel dans une première étape, on mélange à une température supérieure au point de fusion la (les) cire(s) de silicone, éventuellement mélangée(s) à d'autres cires, et on incorpore éventuellement le (les) additif(s) liposoluble(s) ; dans une deuxième étape, on mélange à la phase grasse obtenue la (les) charge(s) solide(s) ; dans une troisième étape, on prépare une phase aqueuse contenant les deux types de polymère(s) acrylique(s) hydrosoluble(s) et, éventuellement d'autres polymères hydrosolubles ainsi que des actifs hydrosolubles ; et, dans une quatrième étape, on ajoute à ladite phase aqueuse la phase grasse obtenue dans la deuxième étape et on disperse par agitation mécanique la phase grasse dans la phase aqueuse.

Les exemples donnés ci-dessous, à titre purement illustratif et non limitatif, permettront de mieux comprendre l'invention.

**EXAMPLE 1 : FOND DE TEINT**

On prépare une composition ayant en poids la formulation suivante :

| | |
|---|---|
| - Polymère carboxyvinylique alkylé vendu sous la dénomination commerciale "PEMULEN TR2" par la société GOODRICH | 0,1 % |
| - Polymère d'acide carboxyvinylique vendu sous la dénomination "CARBOPOL 940" par la société GOODRICH | 0,6 % |
| - Triéthanolamine | 0,8 % |
| - Eau ... qsp | 100 % |
| - Polydiméthylsiloxane-ol dans cyclométhicone vendu sous la dénomination commerciale "Q2-1401" par la société DOW CORNING | 5 % |
| - Alkyl diméthicone vendue sous la dénomination commerciale "ABIL WAX 9810" par la société GOLDSCHMIDT | 4 % |
| - Alkoxydiméthicone vendue sous la dénomination commerciale "ABIL WAX 2440" par la société GOLDSCHMIDT | 11 % |
| - Cyclométhicone vendue par la société DOW CORNING | 5 % |
| - Glycérine | 3 % |
| - Conservateur ... qs | |
| - Dioxyde de titane | 4 % |
| - Oxyde de fer jaune | 1,43 % |
| - Oxyde de fer rouge | 0,55 % |
| - Oxyde de fer noir | 0,22 % |

On obtient un fond de teint cireux, stable au cours du temps, applicable à l'éponge, de texture originale, très douce et mate.

**EXEMPLE 2 : FARD A PAUPIERE**

On prépare une composition ayant en poids la formulation suivante :

| | | |
|---|---|---|
| - Polymère carboxyvinylique alkylé vendu sous la dénomination commerciale "PEMULEN TR2" par la société GOODRICH | 0,1 | % |
| - Copolymère d'acrylate d'ammonium et d'acrylamide vendu sous la dénomination commerciale "PAS 5161" par la société HOECHST | 1,5 | % |
| - Polydiméthylsiloxane vendue sous la dénomination commerciale "Q2-1403" par la société DOW CORNING | 5 | % |
| - Triéthanolamine | 0,1 | % |
| - Dibéhénoxydiméthicone vendu sous la dénomination commerciale "ABIL WAX 2440" par la société GOLDSCHMIDT | 5 | % |
| - Stéaroxydiméthicone vendu sous la dénomination commerciale "VP 1622" par la société WACKER | 10 | % |
| - Glycérine | 5 | % |
| - Pigments | 12,5 | % |
| - Conservateur ................ qs | | |
| - Eau ........................... qsp | 100 | % |

Le fard obtenu est crémeux, stable au cours du temps, s'étale au doigt ou à l'applicateur, est doux et facile à étaler et il a une bonne tenue.

**EXAMPLE 3 : FARD A JOUES**

On prépare une composition ayant en poids la formulation suivante :

| | | |
|---|---|---|
| - Polymère carboxyvinylique alkylé vendu sous la dénomination commerciale "PEMULEN TR2" par la société GOODRICH | 0,1 | % |
| - Copolymère d'acrylate de sodium et d'acrylamide vendu sous la dénomination commerciale "HOSTACERIN" par la société HOECHST | 1,2 | % |
| - Triéthanolamine | 0,1 | % |
| - Stéaroxy diméthicone vendu sous la dénomination commerciale "VP 1621" par la société WACKER | 15 | % |
| - Cyclomethicone | 10 | % |

- Polydiméthylsiloxane vendu sous la dénomination "Q2-1403" par la société DOW CORNING .................. 5 %
- Glycérine ........................................................ 5 %
- Conservateur ................. qs
- Pigments . ........................................................ 7,9 %
- Eau ............................ qsp ............................ 100 %

On obtient un fard très doux, stable au cours du temps, facile à étaler au doigt ou à l'éponge et qui se tient bien.

## Revendications

1. Composition cosmétique pour le maquillage de la peau contenant au moins une cire de silicone, caractérisée par le fait que la (les) cire(s) de silicone est (sont) dispersée(s) dans une phase aqueuse contenant deux types de polymère(s) acrylique(s) hydrosoluble(s), le premier type de polymère(s) étant constitué par au moins un copolymère d'un acide monoéthylénique en $C_3$ - $C_6$ ou de son anhydride avec un ester acrylique à longue chaîne, et le second type de polymère(s) étant constitué par au moins un homo- ou copolymère d'acide acrylique ou un de ses sels, les copolymères d'acide acrylique avec un ester d'acide acrylique à longue chaîne et leurs sels étant exclus.

2. Composition selon la revendication 1, caractérisée par le fait que dans le premier type de polymère(s) acrylique(s), l'acide monomère copolymérisé a pour formule :

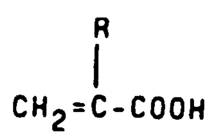

formule dans laquelle R représente H, un halogène, OH, un radical lactone ou lactame, un groupe -C≡N, ou un radical alkyle, aryle, aralkyle, alkylaryle ou cycloaliphatique.

3. Composition selon l'une des revendications 1 ou 2, caractérisée par le fait que l'ester monomère a pour formule :

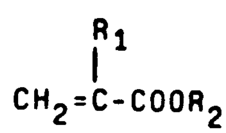

formule dans laquelle :
$R_1$ est H, méthyle ou éthyle et
$R_2$ est un radical alkyle en $C_8$ - $C_{30}$, un radical oxyalkylène en $C_8$ - $C_{30}$, ou un radical carbonyloxyalkylène en $C_8$ - $C_{30}$.

4. Composition selon l'une des revendications 1 à 3, caractérisée par le fait que, dans le copolymère, la proportion d'acide monomère est de 90 à 98 % en poids et la proportion d'ester monomère est de 10 % à 2 % en poids.

5. Composition selon l'une des revendications 1 à 4, caractérisée par le fait que le premier type de polymère(s) acrylique(s) hydrosoluble(s) est présent dans des proportions comprises entre 0,01 et 1 % en poids par rapport au poids total de la composition.

**6.** Composition selon la revendication 5, caractérisée par le fait que le premier type de polymère(s) acrylique(s) hydrosoluble(s) est présent dans des proportions comprises entre 0,02 et 0,2 % en poids.

**7.** Composition selon l'une des revendications 1 à 6, caractérisée par le fait que le second type de polymère acrylique hydrosoluble est un homo- ou copolymère d'acide acrylique réticulé ou un de ses sels.

**8.** Composition selon la revendication 1 ou 7, caractérisée par le fait que le copolymère d'acide acrylique est un copolymère d'un sel d'acide acrylique et acrylamide.

**9.** Composition selon l'une des revendications 1 à 8, caractérisée par le fait que le second type de polymère(s) acrylique(s) hydrosoluble(s) ou un de ses sels est présent dans des proportions comprises entre 0,2 et 2 % en poids par rapport au poids total de la composition.

**10.** Composition selon la revendication 9, caractérisée par le fait que le second type de polymère(s) acrylique(s) ou un de ses sels est présent dans des proportions comprises entre 0,4 et 1,5 % en poids.

**11.** Composition selon l'une des revendications 1 à 9, caractérisée par le fait que la (les) cire(s) de silicone est (sont) un organosiloxane de formule :

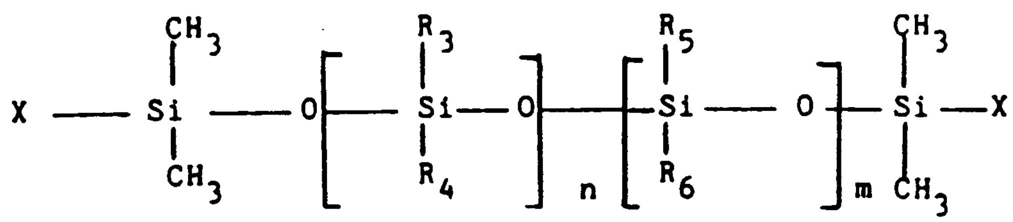

formule dans laquelle :

$R_3$ , $R_5$ et $R_6$ sont, indépendamment les uns des autres, un radical alkyle ou alcoxy en $C_1$ - $C_{30}$ ou un radical aryle en $C_6$ - $C_{30}$ ;

$R_4$ est un radical alkyle ou alcoxy en $C_2$ - $C_{36}$ ou un radical alkyle ou alcoxy en $C_2$ - $C_{36}$ substitué par un ester, $R_4$ pouvant également être un radical méthyle lorsque X est un radical alkyle ou alkoxy en $C_2$ - $C_{30}$ ou un ester en $C_2$ - $C_{30}$ ;

X est un radical alkyle ou alcoxy en $C_1$ - $C_{30}$ , un radical aryle en $C_6$ - $C_{30}$ ou un ester en $C_1$ - $C_{30}$ ;

n est un nombre entier compris entre 1 et 100 ;

m est 0 ou un entier compris entre 1 et 100.

**12.** Composition selon l'une des revendications 1 à 10, caractérisée par le fait que la cire de silicone est un organosiloxane obtenu par action d'une cire naturelle sur un squelette de silicone réactif.

**13.** Composition selon l'une des revendications 1 à 12, caractérisée par le fait que la (ou les) cire(s) de silicone représente(nt) de 10 à 60 % en poids de la composition.

**14.** Composition selon l'une des revendications 1 à 13, caractérisée par le fait que la phase aqueuse de l'émulsion contient au moins un polymère hydrosoluble autre que ceux des deux types de polymère(s) acrylique(s) hydrosoluble(s) définis dans les revendications 1 à 3.

**15.** Composition selon l'une des revendications 1 à 14, caractérisée par le fait que la (ou les) cire(s) de silicone est (ou sont) mélangée(s) avec au moins une cire animale, végétale, minérale ou synthétique, la (les) cire(s) de silicone représentant au moins 20 % en poids par rapport au poids total des cires et au moins 10 % en poids par rapport au poids total de la composition.

**16.** Composition selon l'une des revendications 1 à 15, caractérisée par le fait qu'elle contient des pigments et/ou des charges non colorées.

**17.** Procédé de préparation d'une composition selon les revendications 1 à 16, caractérisé par le fait que, dans une première étape, on mélange, à une température supérieure au point de fusion, la (les) cire(s) de silicone, éventuellement mélangées à d'autres cires et on incorpore éventuellement le (les) additif(s) liposoluble(s) ; dans une deuxième étape, on mélange à la phase grasse obtenue la (les) charge(s) solide(s) ; dans une troisième étape, on prépare une phase aqueuse contenant les deux types de polymère(s) acrylique(s) hydrosoluble(s) et éventuellement d'autres polymères hydrosolubles, ainsi que des actifs hydrosolubles ; et, dans une quatrième étape, on ajoute à ladite phase aqueuse, la phase grasse obtenue dans la deuxième étape et on disperse par agitation mécanique la phase grasse dans la phase aqueuse.

## Claims

**1.** Cosmetic composition for the make-up of the skin, containing at least one silicone wax, characterized in that the silicone wax(es) is (are) dispersed in an aqueous phase containing two types of water-soluble acrylic polymer(s), the first type of polymer(s) consisting of at least one copolymer of a $C_3$-$C_6$ monoethylenic acid or of its anhydride with a long-chain acrylic ester, and the second type of polymer(s) consisting of at least one homo- or copolymer of acrylic acid or one of its salts, the copolymers of acrylic acid with a long-chain acrylic acid ester and their salts being excluded.

**2.** Composition according to Claim 1, characterized in that in the first type of acrylic polymer(s), the copolymerized monomeric acid has the formula:

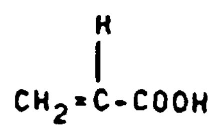

in which formula R denotes H, a halogen, OH, a lactone or lactam radical, a -C=N group or an alkyl, aryl, aralkyl, alkylaryl or cycloaliphatic radical.

**3.** Composition according to either of Claims 1 and 2, characterized in that the monomeric ester has the formula:

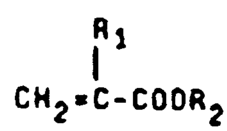

in which formula:
$R_1$ is H, methyl or ethyl and
$R_2$ is a $C_8$-$C_{30}$ alkyl radical, a $C_8$-$C_{30}$ oxyalkylene radical or a $C_8$-$C_{30}$ carbonyloxyalkylene radical.

**4.** Composition according to one of Claims 1 to 3, characterized in that, in the copolymer, the proportion of monomeric acid is from 90 to 98 % by weight and the proportion of monomeric ester is from 10 % to 2 % by weight.

**5.** Composition according to one of Claims 1 to 4, characterized in that the first type of water-soluble acrylic polymer(s) is present in proportions of between 0.01 and 1 % by weight relative to the total weight of the composition.

**6.** Composition according to Claim 5, characterized in that the first type of water-soluble acrylic polymer(s) is present in proportions of between 0.02 and 0.2 % by weight.

**7.** Composition according to one of Claims 1 to 6, characterized in that the second type of water-soluble acrylic polymer is a crosslinked homo- or copolymer of acrylic acid or one of its salts.

8. Composition according to Claim 1 or 7, characterized in that the acrylic acid copolymer is a copolymer of an acrylic acid salt and acrylamide.

9. Composition according to one of Claims 1 to 8, characterized in that the second type of water-soluble acrylic polymer(s) or one of its salts is present in proportions of between 0.2 and 2 % by weight relative to the total weight of the composition.

10. Composition according to Claim 9, characterized in that the second type of acrylic polymer(s) or one of its salts is present in proportions of between 0.4 and 1.5 % by weight.

11. Composition according to one of Claims 1 to 9, characterized in that the silicone wax(es) is (are) an organosiloxane or formula:

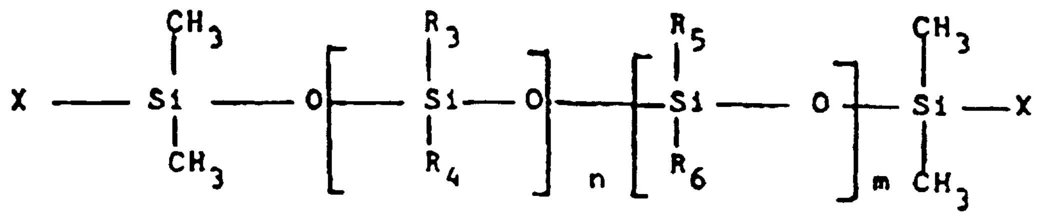

in which formula:

$R_3$, $R_5$ and $R_6$ are, independently of each other, a $C_1$-$C_{30}$ alkyl or alkoxy radical or a $C_6$-$C_{30}$ aryl radical;

$R_4$ is a $C_2$-$C_{36}$ alkyl or alkoxy radical or a $C_2$-$C_{36}$. alkyl or alkoxy radical substituted by an ester, it being also possible for $R_4$ to be a methyl radical when X is a $C_2$-$C_{30}$ alkyl or alkoxy radical or a $C_2$-$C_{30}$ ester;

X is a $C_1$-$C_{30}$ alkyl or alkoxy radical, a $C_6$-$C_{30}$ aryl radical or a $C_1$-$C_{30}$ ester;

n is an integer between 1 and 100;

m is 0 or an integer between 1 and 100.

12. Composition according to one of Claims 1 to 10, characterized in that the silicone wax is an organosiloxane obtained by the action of a natural wax on a reactive silicone backbone.

13. Composition according to one of Claims 1 to 12, characterized in that the silicone wax(es) represents (represent) from 10 to 60 % by weight of the composition.

14. Composition according to one of Claims 1 to 13, characterized in that the aqueous phase of the emulsion contains at least one water-soluble polymer other than those of the two types of water-soluble acrylic polymer(s) defined in Claims 1 to 3.

15. Composition according to one of Claims 1 to 14, characterized in that the silicone wax(es) is (or are) mixed with at least one animal, vegetable, mineral or synthetic wax, the silicone wax(es) representing at least 20 % by weight relative to the total weight of the waxes and at least 10 % by weight relative to the total weight of the composition.

16. Composition according to one of Claims 1 to 15, characterized in that it contains pigments and/or colourless fillers.

17. Process for the preparation of a composition according to Claims 1 to 16, characterized in that, in a first stage, the silicone wax(es), optionally mixed with other waxes, is (are) mixed at a temperature above the melting point and the liposoluble additive(s) is (are) optionally incorporated; in a second stage the fatty phase obtained is mixed with the solid filler(s); in a third stage an aqueous phase is prepared containing the two types of water-soluble acrylic polymer(s) and optionally other water-soluble polymers, as well as water-soluble active substances; and, in a fourth stage, the fatty phase obtained in the second stage is added to the said aqueous phase and the fatty phase is dispersed in the aqueous phase by mechanical agitation.

## Patentansprüche

1. Kosmetisches Mittel zum Schminken der Haut, das wenigstens ein Silikonwachs enthält, dadurch gekennzeichnet, daß das Silikonwachs (die Silikonwachse) in einer wäßrigen Phase dispergiert ist (sind), die zwei Arten eines wasserlöslichen Acrylpolymers (von wasserlöslichen Acrylpolymeren) enthält, wobei die erste Art des Polymers (der Polymere) durch wenigstens ein Copolymer einer monoethylenischen $C_3$-$C_6$-Säure oder eines Anhydrids davon mit einem langkettigen Acrylester gebildet wird und die zweite Art des Polymer (der Polymere) durch wenigstens ein Homo- oder Copolymer von Acrylsäure oder einem ihrer Salze gebildet wird, wobei die Copolymere von Acrylsäure mit einem langkettigen Acrylsäureester und deren Salze ausgeschlossen sind.

2. Mittel nach Anspruch 1, dadurch gekennzeichnet, daß bei der ersten Art des Acrylpolymers (der Acrylpolymere) das copolymerisierte Säuremonomer die Formel:

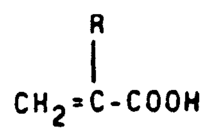

besitzt, worin R für H, ein Halogen, OH, einen Lakton- oder Laktamrest, eine Gruppe -C = N oder einen Alkyl-, Aryl-, Aralkyl-, Alkylarylrest oder cycloaliphatischen Rest steht.

3. Mittel nach einem der Ansprüche 1 oder 2, dadurch gekennzeichnet, daß das Estermonomer die Formel:

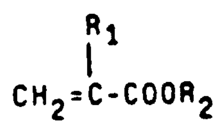

besitzt, worin:

$R_1$ für H, Methyl oder Ethyl steht und

$R_2$ für einen $C_8$-$C_{30}$-Alkylrest, einen $C_8$-$C_{30}$-Oxyalkylenrest oder einen $C_8$-$C_{30}$-Carbonyloxyalkylenrest steht.

4. Mittel nach einem der Ansprüche 1 bis 3, dadurch gekennzeichnet, daß im Copolymer der Anteil des Säuremonomers 90 bis 98 Gew.-% und der Anteil des Estermonomers 10 bis 2 Gew.-% beträgt.

5. Mittel nach einem der Ansprüche 1 bis 4, dadurch gekennzeichnet, daß die erste Art des wasserlöslichen Acrylpolymers (der wasserlöslichen Acrylpolymere) in einem Anteil im Bereich von 0,01 bis 1 Gew.-%, bezogen auf das Gesamtgewicht des Mittels, vorhanden ist.

6. Mittel nach Anspruch 5, dadurch gekennzeichnet, daß die erste Art des wasserlöslichen Acrylpolymers (der wasserlöslichen Acrylpolymere) in einem Anteil im Bereich von 0,02 bis 0,2 Gew.-% vorhanden ist.

7. Mittel nach einem der Ansprüche 1 bis 6, dadurch gekennzeichnet, daß die Zweite Art des wasserlöslichen Acrylpolymers ein vernetztes Homo- oder Copolymer von Acrylsäure oder einem ihrer Salze ist.

8. Mittel nach Anspruch 1 oder 7, dadurch gekennzeichnet, daß das Copolymer von Acrylsäure ein Copolymer aus einem Acrylsäuresalz und Acrylamid ist.

9. Mittel nach einem der Ansprüche 1 bis 8, dadurch gekennzeichnet, daß die zweite Art des wasserlöslichen Acrylpolymers (der wasserlöslichen Acrylpolymere) oder eines der Salze davon in einem Anteil im Bereich von 0,2 bis 2 Gew.-%, bezogen auf das Gesamtgewicht des Mittels, vorhanden ist.

**10.** Mittel nach Anspruch 9, dadurch gekennzeichnet, daß die zweite Art des Acrylpolymers (der Acrylpolymere) oder eines der Salze davon in einem Anteil im Bereich von 0,4 bis 1,5 Gew.-% vorhanden ist.

**11.** Mittel nach einem der Ansprüche 1 bis 9, dadurch gekennzeichnet, daß das Silikonwachs (die Silikonwachse) ein Organosiloxan der Formel:

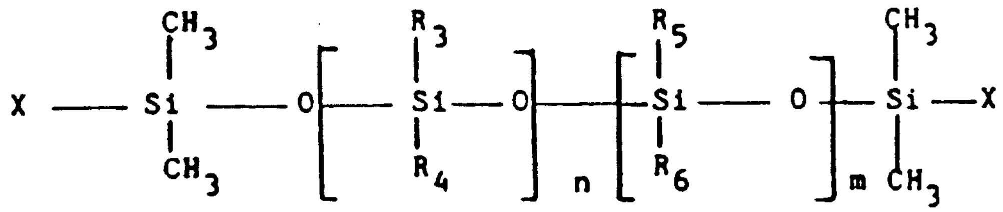

ist (sind), worin:

$R_3$, $R_5$ und $R_6$ unabhängig voneinander für einen $C_1$-$C_{30}$-Alkyl- oder -Alkoxyrest oder einen $C_6$-$C_{30}$-Arylrest stehen;

$R_4$ für einen $C_2$-$C_{36}$-Alkyl- oder -Alkoxyrest oder einen durch einen Ester substituierten $C_2$-$C_{36}$-Alkyl- oder -Alkoxyrest steht, wobei $R_4$ auch einen Methylrest bedeuten kann, wenn X für einen $C_2$-$C_{30}$-Alkyl- oder -Alkoxyrest oder einen $C_2$-$C_{30}$-Ester steht;

X für einen $C_1$-$C_{30}$-Alkyl- oder -Alkoxyrest, einen $C_6$-$C_{30}$-Arylrest oder einen $C_1$-$C_{30}$-Ester steht;

n für eine ganze Zahl von 1 bis 100 steht;

m für 0 oder eine ganze Zahl von 1 bis 100 steht.

**12.** Mittel nach einem der Ansprüche 1 bis 10, dadurch gekennzeichnet, daß das Silikonwachs ein Organosiloxan ist, das durch Einwirkung eines natürlichen Wachses auf ein reaktives Silikongerüst erhalten worden ist.

**13.** Mittel nach einem der Ansprüche 1 bis 12, dadurch gekennzeichnet, daß das Silikonwachs (die Silikonwachse) 10 bis 60 Gew.-% des Mittels ausmacht (ausmachen).

**14.** Mittel nach einem der Ansprüche 1 bis 13, dadurch gekennzeichnet, daß die wäßrige Phase der Emulsion wenigstens ein wasserlösliches Polymer enthält, das verschieden ist von denjenigen der beiden Arten des (der) in den Ansprüchen 1 bis 3 definierten wasserlöslichen Acrylpolymers (Acrylpolymere).

**15.** Mittel nach einem der Ansprüche 1 bis 14, dadurch gekennzeichnet, daß das Silikonwachs (die Silikonwachse) mit wenigstens einem tierischen, pflanzlichen, mineralischem oder synthetischem Wachs vermischt ist (sind), wobei das Silikonwachs (die Silikonwachse) wenigstens 20 Gew.-%, bezogen auf das Gesamtgewicht der Wachse, und wenigstens 10 Gew.-%, bezogen auf das Gesamtgewicht des Mittels, ausmacht (ausmachen).

**16.** Mittel nach einem der Ansprüche 1 bis 15, dadurch gekennzeichnet, daß es Pigmente und/oder ungefärbte Füllstoffe enthält.

**17.** Verfahren zur Herstellung eines Mittels nach einem der Ansprüche 1 bis 16, dadurch gekennzeichnet, daß man in einer ersten Stufe das Silikonwachs (die Silikonwachse) gegebenenfalls in Mischung mit weiteren Wachsen bei einer Temperatur oberhalb des Schmelzpunktes vermischt und gegebenenfalls das fettlösliche Additiv (die fettlöslichen Additive) aufnimmt; in einer zweiten Stufe den festen Füllstoff (die festen Füllstoffe) der erhaltenen Fettphase zumischt; in einer dritten Stufe eine wäßrige Phase herstellt, die die beiden Arten des wasserlöslichen Acrylpolymers (der wasserlöslichen Acrylpolymere) und gegebenenfalls weitere wasserlösliche Polymere sowie die wasserlöslichen Wirkstoffe enthält; und in einer vierten Stufe die in der zweiten Stufe erhaltene Fettphase zu der erwähnten wässrigen Phase gibt und die Fettphase in der wäßrigen Phase durch mechanisches Rühren dispergiert.